# EUROPEAN PATENT APPLICATION

(11) **EP 4 374 831 A1**
(43) Date of publication of application: **29.05.2024**
(21) Application number: 22315296.8
(22) Date of filing: 23.11.2022
(51) Int. Cl.: A61F 2/95

(54) **APPARATUS AND METHOD FOR COMPRESSING A CARDIOVASCULAR IMPLANT**

(71) Applicant: Epygon, 13100 Aix-en-Provence (FR)
(72) Inventor: PECCHIO, Daniele, 13100 Aix-en-Provence (FR); VALERIO, Lorenzo, 13100 Aix-en-Provence (FR); FERRARO, Mauro, 13100 Aix-en-Provence (FR)
(74) Representative: Hepp Wenger Ryffel AG

(57) **Abstract**

A method and apparatus for compressing a cardiovascular implant (40) from a first size to a second smaller size for transcatheter implantation is shown. The apparatus has a compressor mechanism (10) defining, in at least a first operating state, (i) an accommodation region (100) for accommodating the implant in its first size, and (ii) an insertion window (102) for admitting insertion of the implant into the accommodation region. The compression mechanism (10) is configured to compress the implant with respect to a compression axis (16) of the mechanism, and the insertion window (102) is configured to admit insertion of the implant into the accommodation region (100) in an insertion direction that is generally lateral relative to the compression axis (16).

## Description

The present invention relates to the field of cardiovascular implants, more specifically to apparatus and methods for implant compression for loading into a delivery catheter for transcatheter implantation. For example, the implant may be a cardiac implant and/or a prosthetic valve implant (e.g. a prosthetic cardiac valve).

Diseased, damaged, or malformed heart valves often need to be treated with heart valve replacements. This is the case, for example, with valve regurgitation where the damaged valve no longer closes efficiently. The replacement prosthetic cardiac valve will generally be placed by a surgeon or other qualified practitioner either during open-heart surgery or by means of a less invasive method such as a transcatheter intervention. A transcatheter implantation has the potential to be highly beneficial and more efficient, as it allows the delivery of the implant without the use of a heart and lung bypass machine. In this case, the practitioner inserts a catheter through an access point to introduce a valve implant to the delivery site in a compressed condition for implantation.

Although pre-compressed valve implants have been proposed, the most common technique is to compress the implant from its normal size shortly before an intervention, in order to reduce risk of damage to the implant by being held compressed for too long.
The compression of the implant is commonly done by use of a crimper that reduces the circumference of the implant thus allowing the delivery through minimally invasive techniques.

However, compressing an implant, especially a valve implant, is a complicated task requiring at least one, often more than one, trained technician. Some crimpers require components on both axial sides of the implant. Such crimper components have to be manually assembled together around the implant while at the same time inserting the implant axially into the assembly. The need to assemble crimper components together makes the procedure labour intensive, especially for such a preliminary step in the overall procedure. It may increase the risk of human error that could damage the implant. The problem is exacerbated by the crimper components having to be kept sterile at all times so as not to contaminate the implant. The technicians wear surgical gloves that limit their dexterity. It would be more cost-efficient if the crimping operation to be carried out by a single technician, but the above process makes this goal more difficult to achieve.

It may be a non-limiting aim to address and/or mitigate one or more of the above issues.

Aspects of the invention are defined in the claims.

Additionally or alternatively, a first aspect of the invention provides apparatus for compressing a cardiovascular implant from a first (e.g. pre-compressed) size to a second smaller size for transcatheter implantation.

The apparatus can comprise a compressor mechanism defining, in at least a first operating state, (i) an accommodation region for accommodating the implant in its first size, and (ii) an insertion window for admitting insertion of the implant in its first size into the accommodation region.

The compression mechanism is configured to compress the implant with respect to a compression axis of the mechanism. The insertion window is configured to admit insertion of the implant into the accommodation region in an insertion direction that is generally lateral relative to the compression axis.

Provision of an insertion window that permits insertion of the implant in a generally lateral direction can avoid, or at least reduce, the need to manually assemble compressor components axially around the implant while inserting the implant in an axial direction. Instead, components can be pre-assembled in axial relation, and the implant inserted laterally into an accommodation region of the compressor mechanism. This can reduce risk of human error and/or delays and/or potential damage to the implant, and/or it can facilitate use by a single technician.

As used herein, the term "lateral" refers, to a direction that has a substantial component in a direction that is orthogonal to the compression axis, whether or not the insertion direction is strictly orthogonal. Lateral insertion may also be referred to as side-loading and/or insertion from the side and/or top loading and/or loading from the top.

As used herein, the term "compressing" refers to reducing the size of the implant at least in a circumferential direction of the implant, whether or not the implant may increase or decrease in axial length. Depending on the design of the implant, the implant may become longer when compressed circumferentially. Nevertheless, by being compressed circumferentially, the implant can be implanted using a small-diameter catheter to deliver the implant in its compressed configuration.

Also, as used throughout, and in all aspects of the present disclosure, the cardiovascular implant may optionally be a cardiac implant, optionally a prosthetic heart valve. The prosthetic heart valve may optionally be a prosthetic atrioventricular valve such as a mitral valve, and/or optionally be a self-expanding prosthetic heart valve.

In some embodiments, the insertion window extends (e.g. in a circumferential direction) at least partway around the compression axis, optionally only partway around the compression axis.

In some embodiments, the accommodation region is defined at least partly by a body extending axially to one axial side of the accommodation and extending axially to an opposite second axial side of the accommodation region.

In some embodiments, the insertion window is defined by an open lateral side of the compressor mechanism, optionally a top side.

In some embodiments, the compressor mechanism comprise first structure disposed at least partly on one axial side of the accommodation region, and second structure disposed at least partly on an opposite second axial side of the accommodation region, the first structure and the second structure operatively coupled together in said at least one operating state.

Such an arrangement can avoid such structure needing to be manually assembled together while inserting the implant in the mechanism. The first structure and the second structure can be pre-assembled in operative coupled relation.

As used herein, the term "structure" refers to one or more elements or members whether or not connected directly to one another.

Optionally, in an embodiment in which the insertion window extends only partway around the axis, the portions of the first structure and the second structure on opposite axial sides of the accommodation region may be coupled together through a zone not included in the insertion window. Additionally or alternatively, at least a portion of the second structure may be movable relative to at least a portion of the first structure, for example, along a guided path generally parallel to the compression axis.

In some embodiments, the compressor mechanism is configured to translate the implant axially along the compression axis. The first structure may comprise a funnel having an interior surface defining a tapered channel configured for compressing the implant in response to axial translation within the channel. The compressor mechanism may comprise a puller for pulling the implant into and through the channel.

In some embodiments, the second structure may comprise a biasing device for supporting the implant from radially within, for example, to reduce risk of kinking during compression. Optionally, the second structure further comprises a coupling mechanism for moving the biasing device between a retracted position and an extended position. In the retracted position, the biasing device may be positioned not to extend substantially into the accommodation region, so as not to obstruct lateral insertion of the implant into the accommodation region. In the extended position, the biasing device may be position to extend substantially into the accommodation region.

Additionally or alternatively to any of the above, the apparatus may further comprise a carrier configured for supporting the implant prior to compression, for example, in a supplied condition of the implant. The compressor mechanism may be configured for receiving the carrier in the accommodation region. The compressor mechanism may be configured for admitting the carrier into the accommodation region in an insertion direction that is generally lateral with respect to the compression axis.

In some embodiments, the carrier may be axially slidable in the accommodation region, optionally along one or more rails that defined in the accommodation region.

Additionally or alternatively to any of the above, the compressor mechanism may further have one or more of:
(a) a second operating state in which a biasing device is advanced into the accommodation region;
(b) a third operating state in which a biasing device is advanced into an entrance of a compressor funnel of the mechanism, and/or an implant carrier is stopped from travel beyond an entrance of the compressor funnel;
(c) a fourth operating state in which a biasing device is advanced substantially into the interior of a compressor funnel to a stop position; and
(d) a fifth operating state in which a puller is pulled completely through a compressor funnel.

A further closely related aspect of the invention provides apparatus for compressing a cardiovascular implant from a first size to a second smaller size for transcatheter implantation, the apparatus optionally according to the first aspect above, the apparatus comprising a compressor mechanism configured to compress the implant with respect to a compression axis of the mechanism, and a carrier configured for supporting the implant prior to compression,
wherein the apparatus has at least two operating states, optionally at least three operating states, optionally at least four operating states, optionally at least five operating states, said operating states being selected from:
(a) a first operating state in which the compressor mechanism defines (i) an accommodation region for the carrier and the implant, and (ii) an insertion window for admitting insertion of the carrier and the implant into the accommodation region, optionally in an insertion direction that is generally lateral with respect to the compression axis;
(b) a second operating state in which a biasing device is advanced into the accommodation region;
(c) a third operating state in which a biasing device is advanced into an entrance of a compressor funnel of the mechanism, and/or an implant carrier is stopped from travel beyond an entrance of the compressor funnel;
(d) a fourth operating state in which a biasing device is advanced substantially into the interior of a compressor funnel to a stop position; and
(e) a fifth operating state in which a puller is pulled completely through a compressor funnel.

A further closely related aspect of the invention provides a method of compressing a cardiovascular implant from a first size to a second smaller size for transcatheter implantation, the method comprising:
(a) providing a compressor mechanism (10) for compressing the implant with respect to a compression axis.of the compressor mechanism.
(b) inserting the implant into an accommodation region of the compressor mechanism through an insertion window of the compressor mechanism, the step of inserting optionally comprising inserting the implant in an insertion direction that is generally lateral with respect to the compression axis; and
(c) operating the compressor mechanism to compress the implant.

In some embodiments, the step (a) of providing the compressor mechanism may comprise removing the compressor mechanism from packaging to be ready for operative use, or any other manner of making the compressor mechanism available for operative use.

In some embodiments, the insertion step (b) comprises inserting a carrier into the accommodation region, the carrier carrying the implant.

Additionally or alternatively, the method may further comprise a step, between the inserting step (b) and the operating step (c), of manipulating a coupling mechanism to advance a biasing device into an interior of the implant for at least partly supporting the implant from within during compression.

Although certain aspects and features have been highlighted above, this is merely to aid understanding certain principles used herein without limitation. Protection is claimed for any novel idea or feature disclosed herein and/or illustrated in the drawings whether or not emphasis has been placed thereon.

Non-limiting embodiments are now described by way of example, with reference to the accompanying drawings, in which:
Fig. 1 is a schematic perspective side view of a compressor shown in a first operating state, and an implant carrier for insertion into the compressor via a lateral insertion window;
Fig. 2 is a schematic cross-section view from the side, showing the compressor in the first operating state with the implant carrier loaded in an accommodation region;
Fig. 3 is a schematic cross-section similar to Fig. 2, showing the compressor in a second operating state in which a biasing device has been advanced axially to an extended position;
Fig. 4 is a schematic cross-section similar to Fig. 3, showing the compressor in a third operating state in which the implant carrier has been pulled axially towards a compressor funnel;
Fig. 5 is a schematic cross-section similar to Fig. 4, showing the compressor in a fourth operating state in which a puller has been advanced through the funnel;
Fig. 6 is a schematic cross-section similar to Fig. 5, showing the compressor in a fifth operating state in which the implant has been compressed and pulled into a transfer tube;
Fig. 7 is a schematic top view of an example implant in its first expanded configuration;
Fig. 8 is a schematic side view of the implant of Fig. 7;
Fig. 9 is a schematic top view of the implant similar to Fig. 7, but showing the implant in its second compressed configuration;
Fig. 10 is a schematic side view similar to Fig. 8, but showing the implant in its second compressed configuration;
Fig. 11 is a schematic perspective view of the implant in its supplied condition, carried by a holder (omitting tether attachments) .
Fig. 12 is a schematic perspective view of the implant in its supplied condition, showing tether attachments.

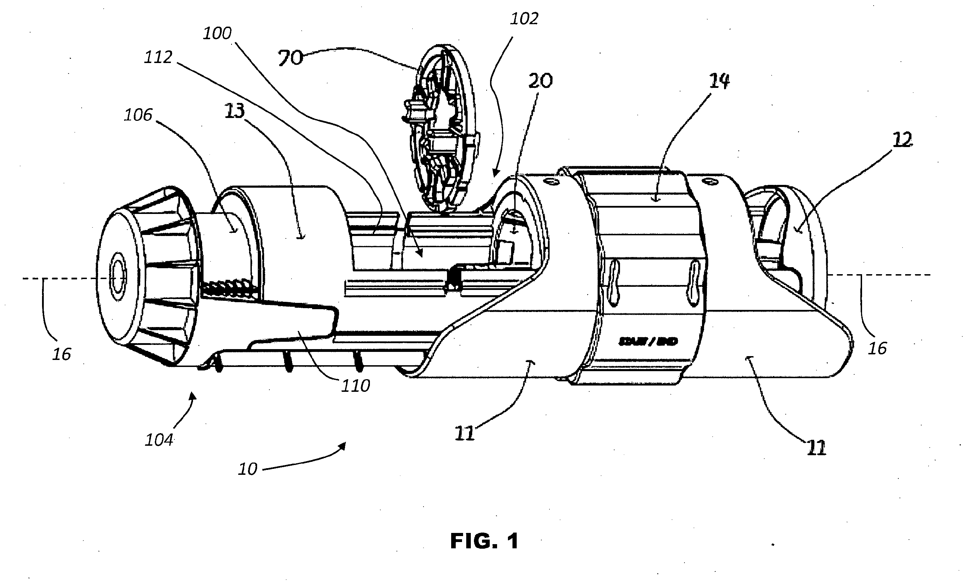

The drawings show apparatus for compressing a cardiovascular implant 40 from a first pre-compression size to a second smaller size for transcatheter implantation. In the different drawings, the same reference numerals are used to denote similar or equivalent features, whether or not described explicitly. In Figs. 1 to 5, as explained later below, the implant 40 is not directly shown, being instead represented by the position of a holder 70 that initially carries the implant 40.

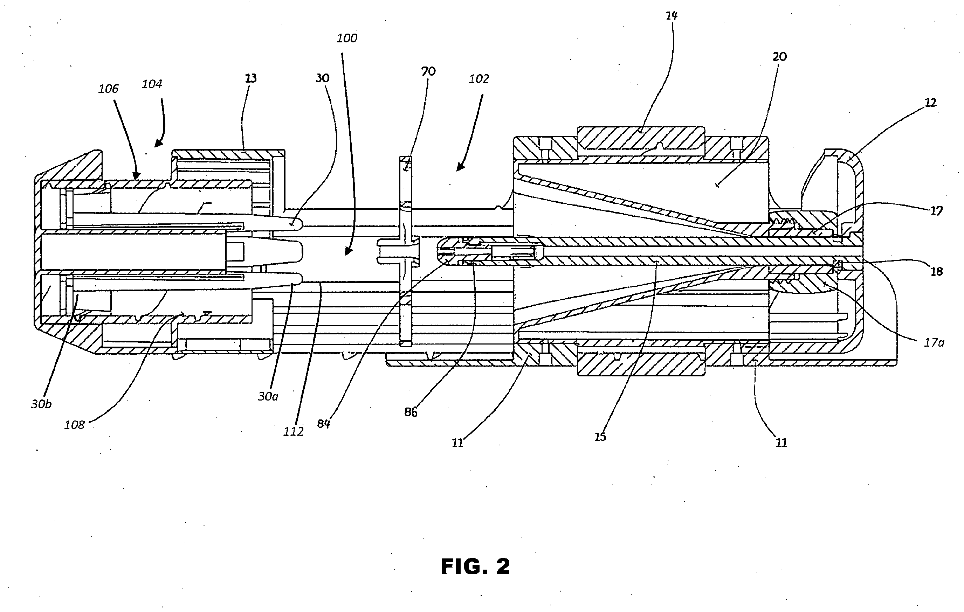

The apparatus comprises a compressor mechanism 10 having components that move relative to each other to define different operating states of the mechanism 10. In at least a first operating state (Figs. 1 and 2), also referred to herein as an insertion state, the mechanism 10 defines an accommodation region 100 for accommodating the implant 40 in its first pre-compression size, and an insertion window 102 for admitting insertion of the implant 40 generally laterally into the accommodation region 100. In the illustrated example, the insertion window 102 is open towards the top of the mechanism 10. The compressor mechanism 10 is operative to compress the implant 40 circumferentially with respect to a compression axis 16, and the insertion window 102 permits the lateral insertion of the implant with respect to the compression axis 16. The insertion window 102 may be defined by an open lateral side (e.g. top side) of the compressor mechanism 10 when in the first operating state. The insertion window 102 may extend at least partway (in the illustrated form only partway) around the compression axis 16.

The compressor mechanism 10 comprises first structure 18a, at least a portion of which is generally disposed on a first axial side of the accommodation region 100, at least in the first operating state. The first structure 18a comprises a funnel 20 having an interior surface defining a tapered channel configured for compressing the implant 40 in response to axial translation within the channel. The funnel 20 has a large entrance aperture communicating with the accommodation region for admitting the implant into the funnel 20 from the accommodation region 100, and a small exit aperture through which the implant exits in its compressed state. The first structure 18a further comprises a puller 15 for pulling the implant into the funnel 20 from the accommodation region 100, and through the funnel, to a transfer tube 17 removably disposed at the funnel exit. The transfer tube 17 is held in position by a locking collar 17a threadedly coupled to the funnel 20.

The puller 15 generally comprises a tubular member having a diameter sufficiently small to fit through the narrow exit aperture of the funnel 20. Movement of the puller 15 is generated by a first carriage 12 to which the puller 15 is coupled, for example, near a first axial end of the compressor mechanism 10. A wall of the carriage 12 is slidably received outside the funnel 20. The carriage wall carries portions of an external thread, to translate the carriage axially with respect to the funnel 20 in response to manual rotation of a threaded actuator ring or knob 14, optionally mounted around the funnel 20. The actuator ring 14 is retained in position by two end-pieces 11 coupled to the funnel 20, the end-pieces defining an external shell of the mechanism that can be held conveniently in one hand.

The compressor mechanism 10 further comprises second structure 18b, at least a portion of which is generally disposed on an opposite second axial side of the accommodation region 100, at least in the first operating state. The second structure 18b comprises a biasing device (also referred to herein as biasing member) 30 for supporting a portion of the implant 40 from radially within as the implant 40 is translated through the funnel 20, to reduce risk of kinking. The biasing member 30 comprises a plurality of cantilever elements 30a extending, from a hub 30b, towards the funnel 20.

The biasing member 30 is coupled to a second movable carriage 13 via a coupling mechanism 104. A wall of the second carriage 13 is slidably received outside the funnel 20. In a similar manner to the first carriage 12, the wall of the second carriage 13 carries portions of an external thread, to translate the second carriage 13 axially with respect to the funnel 20 in response to manual rotation of the threaded actuator ring 14. Therefore, rotation of the actuator ring 14 drives both carriages 12 and 13, generating coordinated translation of the puller 15 and the biasing member 30. Provision of independent carriages enables the puller 15 and the biasing device 30 to move simultaneously (and optionally in unison) over at least a part of the range of movements, but also independently of each other over another part of the range of movements.

The first and second carriages 12 and 13 are shaped to provide the unobstructed insertion window 102 when the mechanism is in the first operating state. At least a portion of at least one, optionally both, carriage(s) defines a respective body which, in the first operating state, extends from one axial side of the accommodation region to an opposite axial side, to at least partly define and/or border the accommodation region.

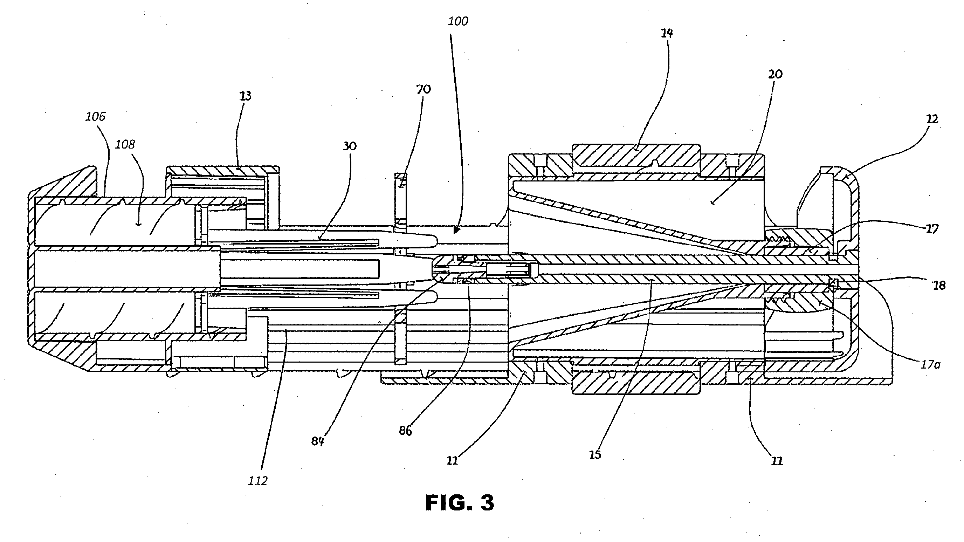

The coupling mechanism 104 additionally enables movement of the biasing member 30 relative to the second carriage 13, between an axially retracted position (Figs. 1 and 2) and an axially advanced and/or axially extended position (Fig. 3). In the retracted position, the biasing member 30 is withdrawn out of the accommodation region 100, to permit insertion of the implant.
In the extended position, the biasing member 30 extends at least partly into the accommodation to engage inside an inserted implant, and provide anti-kinking support.

The coupling mechanism 104 comprises a manually rotatable knob 106 coupled to rotate an intermediate gear 108 that is threadedly coupled to the hub of the biasing member 30. By rotating knob 106, the biasing member 30 can be advanced and positioned in a way that it will support the implant 40 during crimping. Once in position, the biasing member 30 moves together with the implant 40 inside the funnel 20. The biasing member 30 itself is prevented from rotational, movement by a keyed connection to the second carriage 13. Rotation of the knob 106 turns the intermediate gear 108, and the threaded connection converts the rotary motion into axial translation of the biasing member 30 with respect to the second carriage 13. The knob 106 comprises one or more axial lugs 110 engageable with abutment surfaces of the second carriage 13, to define end stops, and a detent lock in different positions of the knob 106. The coupling mechanism 104 is positioned and configured not to obstruct the insertion window 102 in the first operating state.

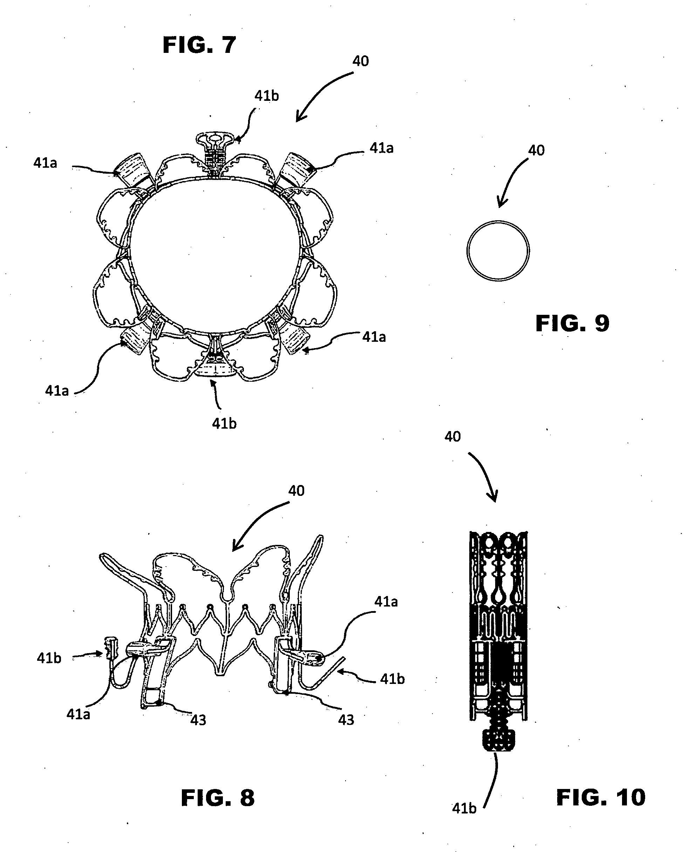

Referring to Figs. 7-12, the implant 40 to be compressed is illustrated, by way of example only, in the form of a self-expanding prosthetic mitral valve. Only the self-expanding frame of the implant is shown, to avoid obscuring the drawings. The implant further comprises a valve component (not shown) comprising flexible material, for example, flexible biological tissue, optionally, pericardial tissue.

Figs. 7, 8, 11 and 12 depict the implant 40 in its pre-compression, first configuration in which the implant is initially supplied. Figs. 9 and 10 depict the implant 40 in its compressed configuration after passing through the funnel 20. The illustrated implant has several operative design features that make the implant awkward to compress. In the first configuration, the implant 40 has a non-circular (e.g. D-shaped) profile that has to be controllably compressed into a round shape. Additionally, the implant 40 has an anchoring system comprising at least one, in the illustrated example six, protuberances 41 (41a, 41b). In the first configuration, the protuberances 41 project outwardly with respect to the implant frame. Four side protuberances 41a are bent radially outwardly, and two end protuberances 41b are folded over on themselves outside the frame. The side protuberances 41a have to be controllably folded inwardly into windows 43 in the frame. As well as the difficulty of folding the side-protuberances 41a, the windows 43 define non-compressible zones in the frame, which results in nonuniform stresses when the frame is compressed circumferentially. The end protuberances 41b have to be controllably bent through a large obtuse angle to extend axially away from the frame. Performing such manipulations, while smoothly compressing the frame circumferentially to a small round size, requires complex.interactions with the compressor apparatus.

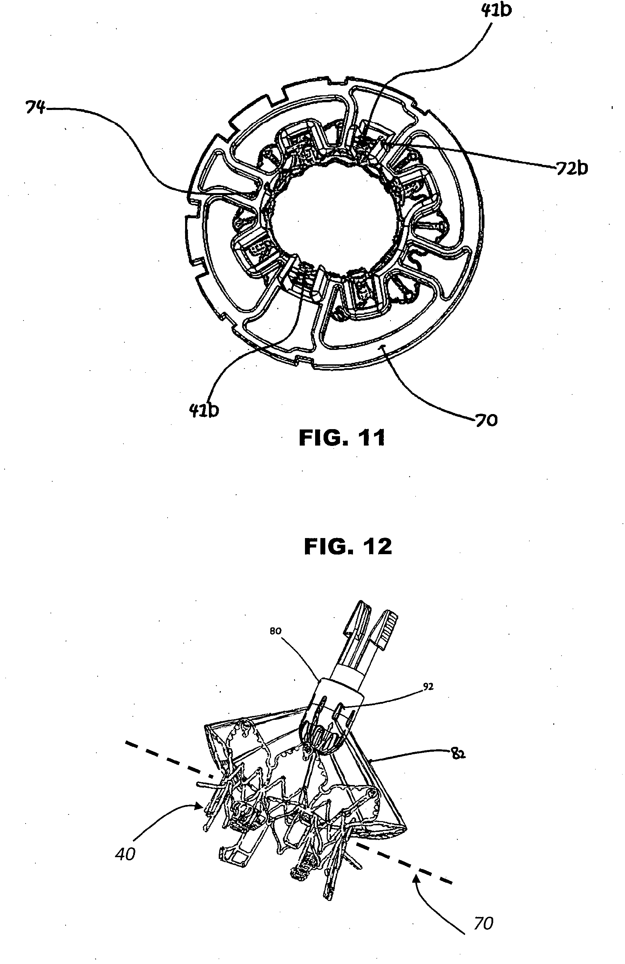

In the illustrated example, the implant 40 is initially supported by a carrier 70 that at least partly circumscribes the implant 40. The carrier 70 and implant 40 are shown together in Fig. 11, and the carrier is depicted schematically in Fig. 12. Generally, in Figs. 1 to 5, only the positions of the carrier 70, and the tether connector 80 (82, 84) are depicted, to avoid obscuring details of the compressor mechanism.

The carrier 70 serves multiple purposes. The carrier 70 can support and protect the implant 40 in its packaged condition prior to use. The carrier 70 can also provide a convenient way of handling the implant 40 when removed from its packaging, without the technician having to touch the implant directly.
The carrier 70 also provides a convenient way of placing the implant 40 into the compressor apparatus 10. The carrier 70 can also function to assist the compression of the implant 40, in particular, the end protuberances 41b. The carrier 70 comprises abutments 72 around which the end protuberances 41b hook, to clip the implant 40 into the carrier 70. Although the carrier 70 provides technical advantages and improves convenience, it is not essential in all embodiments. For example, in an alternative embodiment, the implant 40 could be placed manually into the accommodation region 100 without any carrier.

Fig. 12 also illustrates the implant 40 being initially coupled to one or more tethers 82 extending between the frame and a puller-connector 80, optionally made in two parts 84 and 86. The tethers extend through apertures 92 in the connector 80. The tethers 82 are pre-attached to the frame in the supplied condition of the implant 40. The connector 80 serves to form a connection, for example, a snap-fit connection, to the puller 15 of the compressor mechanism 10, to facilitate pulling of the implant 40 via the tethers 82. In Figs. 1 to 5, only the position of the connector 80 (82, 84) is shown, without the tethers, in order to avoid obscuring other features of the compressor mechanism 10. Although the tethers 82 provide a very convenient way of applying pulling forces controllably to the implant 40 while the implant is being compressed, tethers are not essential in all embodiments. For example, a mechanical engagement could be, used instead to pull or push the implant 40 to translate it into, and through, the funnel 20.

In use, the compressor mechanism 10 is initially placed into the first (e.g. insertion) operating state, in which the second carriage 13 is in its open condition, exposing the accommodation region 100, and the open insertion window 102. The first carriage 12 places the puller 15 so that it is presented near or at the large entrance aperture of the funnel 20, and accessible in the accommodation region 100. Also in the first operating state, the coupling mechanism 104 is set to place the biasing member 30 in its retracted position, so that it does not obstruct the accommodation region 100. In this operating state, the different components of the compressor mechanism 10 remain pre-assembled together, so that the technician does not have to manually assemble the compressor components around the implant 40.

The implant 40 is placed into the accommodation region 100 laterally (e.g. downwardly, from the top) via the insertion window 102, in this example using the carrier 70. Portions of the first carriage 12 and/or the second carriage 13 that are exposed on the inner surface of the accommodation region 100 have axially extending rails 112 that project inwardly from the carriage surface. The outer periphery of the carrier 70 includes notches 114 configured to key with the rails. The keying engagement provides alignment of the carrier 70 with respect to the compressor mechanism 10 around the compression axis. This can enable the implant 40 to be reliably aligned with features of funnel 20 dedicated to compressing the implant 20. For example, the funnel 20 may define, at least at the entrance, a D-shape of the channel to match the non-round shape of the implant when correctly aligned. The funnel 20 may also comprise one or more longitudinal grooves configured for folding the protuberances 41 (e.g. side protuberances 41a) into coincidence with the wall of the frame when correctly aligned.

The keying engagement also permits axial sliding movement of the carrier 70 within the accommodation region 100. This'is useful to permit the carrier 70 to be slid axially into a position in which the puller-connector 80 can be connected to the puller 15 exposed at the entrance to the funnel 20. Axial sliding movement of the carrier 70 is also used as part of the operation of the compressor mechanism 10 as described later below.

Referring to Fig. 3, a next step of the procedure is to manually rotate knob 106 of the coupling mechanism 104 to advance the biasing member 30 axially towards the carrier 70 and implant 14, to arrive at the second operating.state. In the second operating state, the tips of the cantilever fingers of the biasing member 30 are positioned to be inside the carrier 70 and/or inside the implant 40, to provide support from within, and reduce risk of damage to the implant 40 through kinking.

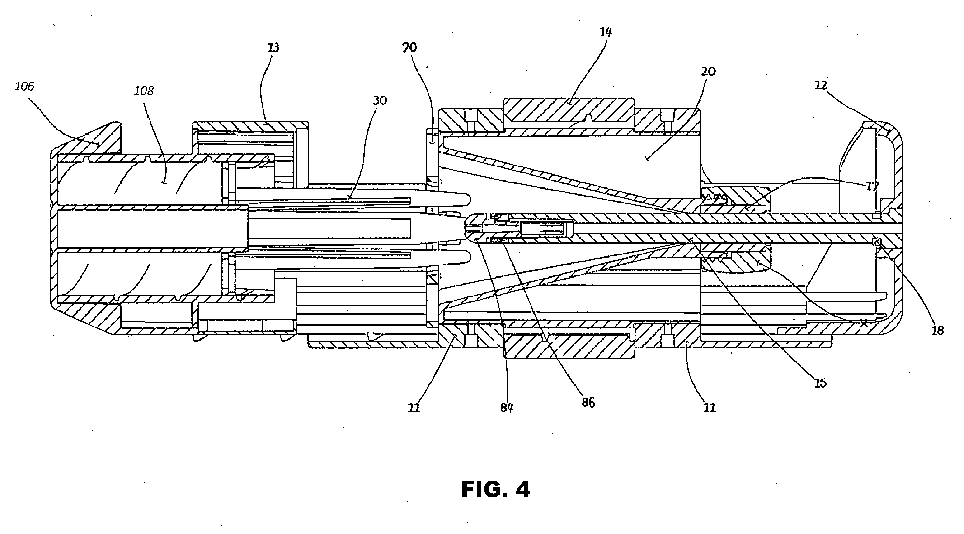

Referring to Fig. 4, a next step of the procedure is to manually rotate the actuator ring 14, to drive movement of the first and second carriages 12 and 13. During this phase of operation, the puller 15 is moved simultaneously, optionally in unison, with the biasing device 30. The puller 15 pulls the implant 40 via the tethers 82, towards the funnel 20.

The carrier 70 slides axially freely along the rails 112 following movement of the implant 40 supported within the carrier 70, so that the carrier 70 and the implant 70 remain coupled together as a unit. The carrier 70 is able to slide towards the funnel 20 until the carrier 70 reaches a stop position in a third operating state of the mechanism, depicted in Fig. 4. In this example, the stop position is defined by the carrier 70 abutting against an end of the funnel 20. Also in the third operating state, a portion of the implant 40 facing towards the funnel 20 may already begin to enter the funnel 20 and begin compression by the surface of the tapered channel.

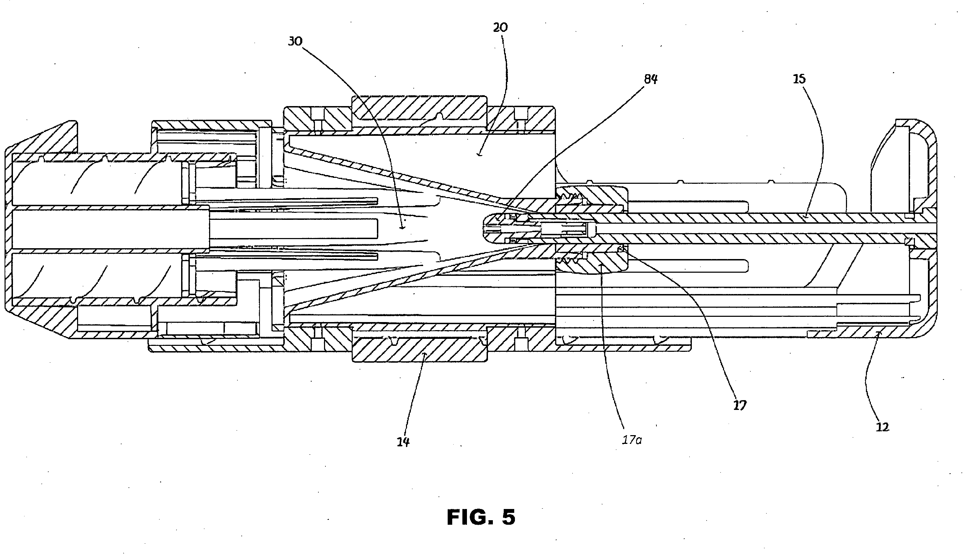

Referring to Fig. 5, continued rotation of the actuator ring 14 continues to drive movement of the first and second carriages 12 and 13, while further movement of the carrier 70 is blocked beyond its stop position. The puller 15 pulls the implant further into the funnel 20, causing the implant 40 to translate axially out of the carrier 70. Axial translation of the implant 40 with respect to the carrier 70 causes the end protuberances 41b to bear against the abutments 72, thereby forcing the end protuberances 41b to unfold or uncurl into an axially straight configuration. The implant 40 enters the funnel 20, which constrains the end protuberances 41b in their unfolded straight configuration.

At the same time as the first carriage 12 moves the puller 15, the second carriage 13 advances the biasing member 30, to maintain support for the implant 40 from radially withing the implant, thereby reducing risk of kinking as the implant is translated through the channel. The second carriage 13 is movable until the biasing member 30 reaches a fourth operating state (depicted in Fig. 5), in which the implant 40 will have been compressed to a sufficiently small size that there is no longer room in the interior of the implant 40 for the biasing member 30. In fourth operating state, further movement of the second carriage 13 is blocked and/or the second carriage 13 disengages from the actuator ring 14.

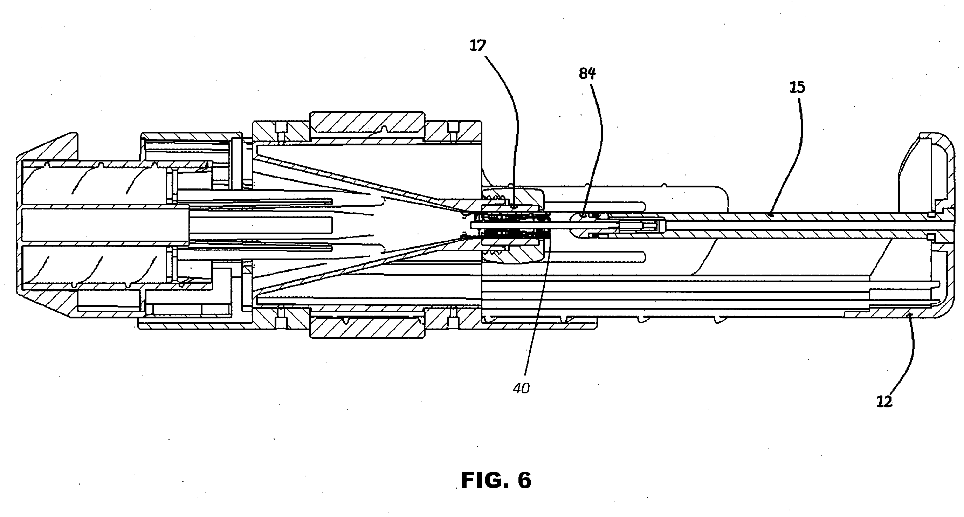

Thereafter, continued rotation of the actuator ring 14 continues to drive movement of the first carriage 12 only, to translate the implant 40 into the narrowest region of the funnel 20, and into the transfer tube 17, reaching a fifth operating state as shown in Fig. 6. Thereafter, the puller 15 can be disconnected from the implant 40, and the locking nut 17a disengaged to release the transfer tube 17 containing the compressed implant 40. The transfer tube 17 facilitates loading of the compressed implant 40 into a delivery catheter (not shown) for transcatheter implantation of the implant.

The techniques described herein, including insertion of the implant in a generally lateral direction relative to a compression axis, can enable a relatively complex compressor mechanism to be provided, without having to manually assemble together component parts axially around the implant. It also facilitates use by a single technician, also referred to as single-operator use.

It will be appreciated that the foregoing description is merely illustrative of a preferred embodiment of the invention, and that many modifications and equivalents may be used within the scope and/or principles of the disclosure.

## Claims

1. Apparatus for compressing a cardiovascular implant (40) from a first size to a second smaller size for transcatheter implantation, the apparatus comprising:
a compressor mechanism (10) defining, in at least a first operating state, (i) an accommodation region (100) for accommodating the implant in its first size, and (ii) an insertion window (102) for admitting insertion of the implant into the accommodation region;
wherein the compression mechanism (10) is configured to compress the implant with respect to a compression axis (16) of the mechanism, and the insertion window (102) is configured to admit insertion of the implant into the accommodation region (100) in an insertion direction that is generally lateral relative to the compression axis (16).

2. Apparatus according to claim 1, wherein the insertion window (102) extends only partway around the compression axis (16) .

3. Apparatus according to claim 1 or 2, wherein the insertion window (102) is defined by an open lateral side of the compressor mechanism (10), optionally a top side.

4. Apparatus according to claim 1, 2 or 3, wherein the compressor mechanism comprises first structure (18a) disposed at least partly on one axial side of the accommodation region (100), and second structure (18b) disposed at least partly on an opposite second axial side of the accommodation region, the first structure and the second structure operatively coupled together in said first operating state.

5. Apparatus according to claim 4, wherein the first structure (18a) comprises a funnel (20) defining a tapered channel configured for compressing the implant as the implant is translated through the funnel.

6. Apparatus according to claim 5, wherein the first structure (18a) further comprises a first carriage (12) that is axially movable with respect to the funnel (20), and a puller (15) coupled to the carriage (12)- for axially translating the implant.

7. Apparatus according to claim 4, 5 or 6, wherein the second structure (18b) comprises a second carriage (13) that is axially movable with respect to the funnel (20), and a biasing device (30) coupled to the second carriage (13) for supporting the implant from radially within the implant.

8. Apparatus according to claim 4, wherein the second structure (18b) further comprises a coupling mechanism (104) for movably coupling the biasing device (30) to the second carriage (13), the coupling mechanism (104) configured to move the biasing member between a retracted position and an extended position with respect to the second carriage.

9. Apparatus according to claim 8, wherein in the retracted position, the biasing device (30) does not extend substantially into the accommodation region (100), and in the extended position, the biasing device (30) extends substantially into the accommodation region (100).

10. Apparatus according to any preceding claim, further comprising a carrier (70) configured for supporting the implant (40) prior to compression, the compressor mechanism (10) configured for receiving the carrier (70) in the accommodation region (100).

11. Apparatus according to claim 10, wherein the compressor mechanism is configured for keyed engagement with the carrier (70), to define at least one discrete insertion orientation of the carrier with respect to the compressor mechanism.

12. Apparatus according to claim 11, wherein the carrier (70) is axially slidable in the accommodation region (100), optionally along one or more rails defined in the accommodation region.

13. Apparatus according to any preceding claim, wherein the compressor mechanism has one or more of:
(a) a second operating state in which a biasing device (30) is advanced into the accommodation region (100);
(b) a third operating state in which a biasing device (30) is advanced into an entrance of a compressor funnel (20) of the mechanism, and/or an implant carrier (70) is stopped from travel beyond an entrance of the compressor funnel (20);
(c) a fourth operating state in which a biasing device (30) is advanced substantially into the interior of a compressor funnel (20) to a stop position; and
(d) a fifth operating state in which a puller (15) is pulled completely through a compressor funnel (20).

14. Apparatus according to any preceding claim, and an implant (40) for compression by the apparatus.

15. A method of compressing a cardiovascular implant from a first size to a second smaller size for transcatheter implantation, the method comprising:
(a) providing a compressor mechanism (10) for compressing the implant with respect to a compression axis of the compressor mechanism.
(b) inserting the implant into an accommodation region of the compressor mechanism through an insertion window of the compressor mechanism, the step of inserting comprising inserting the implant in an insertion direction that is generally lateral with respect to the compression axis; and
(c) operating the compressor mechanism to compress the implant.

16. A method according to claim 15, wherein the insertion step (b) comprises inserting a carrier into the accommodation region, the carrier carrying the implant.

17. A method according to claim 15 or 16, further comprising a step, between the inserting step (b) and the operating step (c), of manipulating a coupling mechanism to advance a biasing device into an interior of the implant for at least partly supporting the implant from within during compression.

18. A method according to claim 15, 16 or 17, wherein the operating step (c) comprising manually operating an actuator to translate the implant axially from the accommodation region and through a funnel of the compressor mechanism.
